# EUROPEAN PATENT SPECIFICATION

(11) **EP 1 541 554 B1**
(45) Date of publication and mention of the grant of the patent: **03.12.2008**
(21) Application number: 03794249.7
(22) Date of filing: 05.09.2003
(51) Int. Cl.: C07D 209/08, A61K 31/404, A61K 9/20, A61K 9/48, A61K 47/02, A61K 45/00, A61P 13/02

(54) **CRYSTAL FOR ORAL SOLID DRUG AND ORAL SOLID DRUG FOR DYSURIA TREATMENT CONTAINING THE SAME**
KRISTALL FÜR EIN ORALES, FESTES ARZNEIMITTEL, UND EIN ORALES, FESTES ARNZEIMITTEL ZUR BEHANDLUNG VON DYSURIE, DAS DIESEN ENTHÄLT
CRISTAL POUR MEDICAMENT SOLIDE A ADMINISTRATION ORALE ET MEDICAMENT SOLIDE A ADMINISTRATION ORALE DESTINE AU TRAITEMENT DE LA DYSURIE CONTENANT CE CRISTAL

(30) Priority: 06.09.2002 JP 2002262157
(43) Date of publication of application: 15.06.2005
(73) Proprietor: Kissei Pharmaceutical Co., Ltd., Matsumoto-City Nagano-Pref. 399-8710 (JP)
(72) Inventor: TSURU, Eiji, c/o Central Res. Laboratories, Hotaka-machi, Minamiazumi-gun, Nagano (JP); TODA, Michio, c/o Central Research Laboratories, Hotaka-machi, Minamiazumi-gun, Nagano (JP); HIRATA, Kazuma, c/o Central Research Laboratories, Hotaka-machi, Minamiazumi-gun, Nagano (JP)
(74) Representative: Hayes, Adrian Chetwynd
(86) International application number: PCT/JP2003/011345
(87) International publication number: WO 2004/022538

(56) References cited:
- WO-A-99/48530
- WO-A1-99/15202
- JP-A- 7 330 726
- JP-A- 2000 247 998
- JP-A- 2002 265 444

## Description

### TECHNICAL FIELD

The present invention relates to a crystal for an oral solid medicament. More particularly, the present invention relates to a crystal as specified in the claims for an oral solid medicament of an indoline compound represented by the formula (hereinafter referred to as KMD-3213): 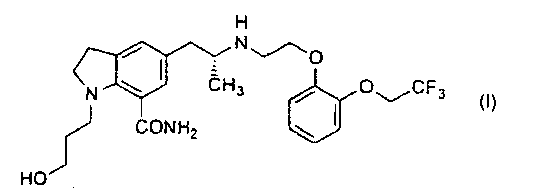 which exerts an α₁-adrenoceptor (α₁-AR) blocking effect and is useful as a therapeutic agent for dysuria, and an oral solid medicament for dysuria treatment comprising the crystal as an active ingredient.

The invention also relates to an oral solid medicament which comprises as active ingredients a crystal of KMD-3213 for an oral solid medicament and at least one member selected from a group of an α₁-AR blocking agent except for KMD-3213, an anticholinergic agent, a 5α-reductase inhibitor, a sex hormone agent, an antianxiety agent, a cholinergic agent, a cholinesterase inhibitor, an antiinflammatory agent and an antibacterial agent.

Furthermore, the present invention relates to a medicament for dysuria treatment which comprises combining a medicament comprising as an active ingredient a crystal as specified in the claims for an oral solid medicament of KMD-3213 with a medicament comprising at least one member selected from a group of an α₁-AR blocking agent except for KMD-3213, an anticholinergic agent, a 5α-reductase inhibitor, a sex hormone agent, an antianxiety agent, a cholinergic agent, a cholinesterase inhibitor, an antiinflammatory agent and an antibacterial agent.

### BACKGROUND ART

It is known that KMD-3213 comprised as an active ingredient in the oral solid medicament for dysuria treatment has a selective suppression effect of the urethra smooth muscle contraction and is an extremely useful compound as the medicament for dysuria treatment which does not cause a strong hypotensive effect or orthostatic hypotension. However, its concrete detailed preparing method and purification method have not been reported. Furthermore, physical properties of KMD-3213 have been reported on data of IR (Infra Red Absorption spectrum), specific rotation and NMR (Nuclear Magnetic Resonance spectrum), but its appearances and crystalline polymorphs have not been reported. (see the following Literature 1)

That is, up to the present, as for crystalline polymorphs of KMD-3213, any concrete preparing method has not been reported. In addition, there are not any report or suggestion concerning what types of crystal forms exist, these preparing methods, these properties etc.

On the other hand, as for pharmaceutical compositions comprising as an active ingredient KMD-3213 or its pharmaceutically acceptable salt, or pharmaceutically acceptable solvate thereof, these dosage forms have been exemplified as the general description concerning whole compounds represented by certain general formula including KMD-3213. These compositions are only described as producible according to general pharmaceutical methods (see the following Literature 1). Furthermore, in the general description as to pharmaceutical compositions for the treatment of lower urinary tract disorders comprising as an active ingredient an α₁-AR blocker including KMD-3213, oral solid dosage forms are exemplified. And, it is described that preferable preparations are a continuous release type of sustained release dosage forms, and that the preparations are producible according to known methods together with exemplifying examples of pharmaceutical additives (see the following Literature 2).

That is, up to the present, preferable crystal forms like the present invention of KMD-3213 for a solid medicament and an oral solid medicament for dysuria comprising the same are not reported nor suggested at all.
Literature 1: Japanese unexamined publication H06-220015
Literature 2: Japanese unexamined publication 2001-288115

### DISCLOSURE OF THE INVENTION

The present invention provides a crystal as specified in the claims for an oral solid medicament of KMD-3213 which is extremely useful as a therapeutic agent for dysuria with less effect on blood pressure and an oral solid medicament for dysuria treatment comprising the same.

The present invention also provides a medicament for dysuria treatment which comprises as active ingredients KMD-3213 as specified in the claims and at least one member selected from a group of an α₁-AR blocking agent except for KMD-3213, an anticholinergic agent, a 5α-reductase inhibitor, a sex hormone agent, an antianxiety agent, a cholinergic agent, a cholinesterase inhibitor, an antiinflammatory agent and an antibacterial agent.

Furthermore, the present invention provides a medicament for dysuria treatment which comprises combining a medicament comprising as an active ingredient a crystal as specified in the claims for an oral solid medicament of KMD-3213 with a medicament comprising as an active ingredient at least one member selected from a group of an α₁-AR blocking agent except for KMD-3213, an anticholinergic agent, a 5α-reductase inhibitor, a sex hormone agent, an antianxiety agent, a cholinergic agent, a cholinesterase inhibitor, an antiinflammatory agent and an antibacterial agent.

### BRIEF DESCRIPTION OF THE DRAWINGS

Fig. 1 is a graph showing an X-ray powder diffraction pattern of crystal form α of KMD-3213. The ordinate shows the X-ray intensity in Kcps and the abscissa shows 2θ(°).
Fig. 2 is a graph showing an X-ray powder diffraction pattern of crystal form β of KMD-3213. The ordinate shows the X-ray intensity in Kcps and the abscissa shows 2θ(°).
Fig. 3 is a graph showing an X-ray powder diffraction pattern of crystal form γ of KMD-3213. The ordinate shows the X-ray intensity in Kcps and the abscissa shows 2θ(°).

### BEST MODE FOR CARRYING OUT THE INVENTION

On the occasion of the development for dysuria treatment of oral solid medicaments comprising as an active ingredient KMD-3213 which is extremely useful for dysuria treatment, the present inventors conducted extensive investigation of preferable crystal forms of KMD-3213 for the oral solid medicaments. The present inventors have found a novel crystal form suitable for oral solid medicaments. Based on the founding, the present invention has been accomplished.

It is required that a medicament for treatment always shows a constant action and effect. For that purpose, the content of an active ingredient must be substantially the same in a medicament. In such a case, the amount of crystal solvent or residual solvent such as adhesion solvent is important as well as the stability of the active ingredient. Concerning an oral solid medicament, solubility and specific volume of the active ingredient are also important factors.

Generally, an amorphous compound is often unsuitable for using for oral solid medicaments even though it shows better solubility, because its stability is bad, its specific volume is variable and it is easy to adhere solvent or to absorb moisture. On the other hand, a crystalline compound has lower solubility, but is stable and is less subject to incompatible combination with excipients or to change by time. In addition, since it is not hygroscopic and its specific volume is likely constant, it is easy to control the amounts of the active ingredient. Therefore, a crystal active ingredient is preferable for an oral solid medicament so long as the crystal has no problem about its solubility.

Besides, some compounds may have plural crystal forms, that is, polymorphism. Each crystal form may show different solubility or stability, and the differences may affect the hygroscopicity or pharmacodynamics. Therefore, in case that polymorphism exists in an active ingredient used for medicaments, confirmation of characteristics of its polymorphism used for medicament is required.

The present inventors had earnestly investigated polymorphism of KMD-3213 which is the most useful for dysuria treatment among the indoline compounds described in the above Literature 1. As a result, the inventors have found that there are at least three crystal forms and one of them is preferable to use for an oral solid medicament.

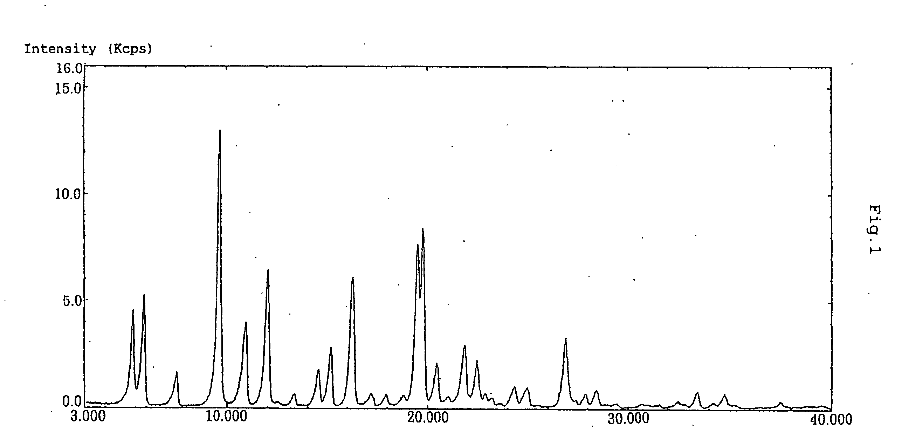

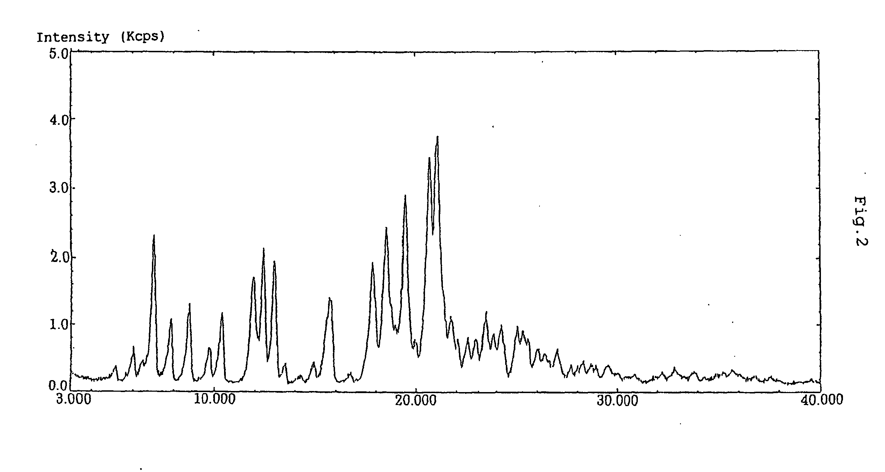

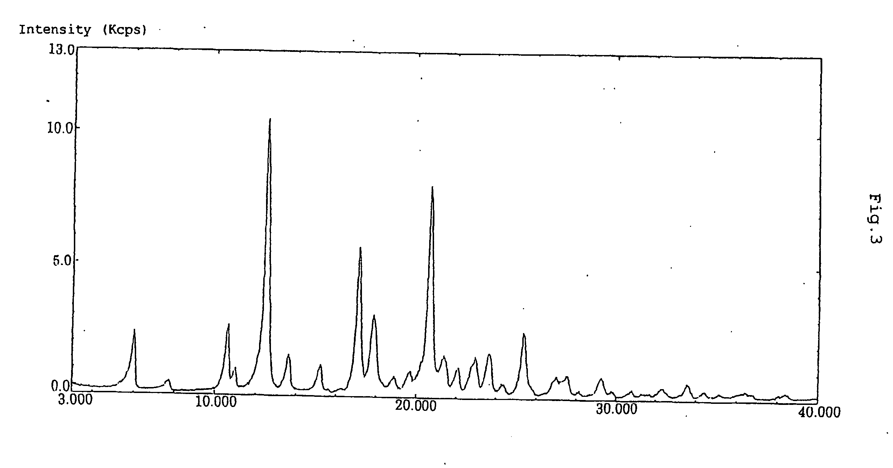

That is, KMD-3213 has at least three crystal forms shown by the powder X-ray diffraction patterns in Fig. 1 to Fig. 3. Concretely, the present inventors have found that there are three crystal forms, (1) a crystal characterized by main peaks of 5.5° ± 0.2°, 6.1° ± 0.2°, 9.8° ± 0.2°, 11.1° ± 0.2°, 12.2° ± 0.2°, 16.4° ± 0.2°, 19.7° ± 0.2° and 20.0° ± 0.2° as 2θ (hereinafter referred to as crystal form α); (2) a crystal characterized by main peaks of 7.0° ± 0.2°, 12.5° ± 0.2°, 18.5° ± 0.2°, 19.5° ± 0.2°, 20.7° ± 0.2°and 21.1° ± 0.2° as 2θ (hereinafter referred to as crystal form β); and (3) a crystal characterized by main peaks of 6.0° ± 0.2°, 10.6° ± 0.2°, 12.6° ± 0.2°, 17.1° ± 0.2°, 17.9° ± 0.2°, 20.7° ± 0.2° and 23.7° ± 0.2°as 2θ (hereinafter referred to as crystal form γ). These crystal forms can be prepared as follows.

The crystal form α can be prepared by dissolving crude crystals thereof in an appropriate amount of ethyl acetate, ethyl formate, acetone, methyl ethyl ketone, acetonitrile, tetrahydrofuran or a mixed solvent of acetone and acetonitrile (1:1) and so on, preferably ethyl acetate under heating, allowing to stand at room temperature, and making crystal precipitates gradually.

The crystal form β can be prepared by dissolving crude crystals thereof in an appropriate amount of methanol under heating, adding petroleum as a poor solvent, stirring the mixture vigorously, and making crystal precipitates forcibly and suddenly. The crystal form β can be also prepared by dissolving crude crystal thereof in ethanol or 1-propanol, and cooling quickly.

The crystal form γ can be prepared by dissolving crude crystal thereof in an appropriate amount of toluene, a mixed solvent of acetonitrile and toluene (1:4) or a mixed solvent of ethyl acetate and toluene (1:19), preferably toluene under heating, allowing to stand at room temperature, and making crystal precipitates gradually. The crystal form γ can be also prepared by dissolving crude crystal thereof in 2-propanol, and adding an appropriate amount of toluene thereto to precipitate a crystal.

Stability and hygroscopicity of each crystal form prepared as the above were measured. As a result, it is confirmed that the hygroscopicities of these three crystal forms are better than that of an amorphous form obtained by dissolving the crystals in dioxane and freezing and drying the solution and that there are little difference among them. Furthermore, it is also confirmed that the stabilities of these three crystal forms do not make much difference, and however that the crystal form α is white with almost no coloration in the appearance and the most stable. Accordingly, the present inventors found that the crystal form α is the best for the crystal for an oral solid medicament as regards stability and hygroscopicity.

Furthermore, among the crystal forms of the above KMD-3213, the crystal form β has a manufacturing issue in industrial preparation, since it is prepared by adding a poor solvent into a warmed solution to make crystal precipitates forcibly and suddenly as described above. For example, apparatuses for the industrial preparation become big and it may be difficult to get constant quality of the crystal. In case that the crystal form β is prepared by dissolving in ethanol or 1-propanol, and cooling quickly, it has another issue that the yields and purity tend to be irregular because different crystal forms are easy to mix therewith depending on the cooling speed, the temperature, the degree of stirring and the like.

The crystal form γ is prepared by cooling the warmed solution, allowing the crystal to precipitate gradually and unforcibly according to the usual recrystallization method, so the industrial apparatus can be small and it is easy to prepare a uniform crystal by controlling quantity of solvent, heating temperature, cooling temperature, cooling speed and the like. Therefore, there is no issue in the industrial preparation. However, the recrystallization solvent of this crystal form γ is toluene or a mixed solvent comprised mainly of toluene. Accordingly, the crystal form γ has a problem that it takes a lot of trouble to remove the solvent and it is comparatively difficult to completely remove the residual solvent because of toluene's high boiling point. As for a residual solvent in a raw material for a medicament, an upper limit of quantity has been determined depending on the kind of solvent. The amount of toluene is required to be not more than 890 ppm. For that reason, the crystal form γ has a problem at this point.

On the other hand, the crystal form α has no problem in respect of industrial preparation as the crystal form β and can be prepared regularly, easily and in a large scale, in addition, without a problem of the residual solvent like crystal form γ. For that reason, the crystal form α is the most preferable for the oral solid medicament at points of industrial preparation and quality.

Accordingly, stable oral solid medicaments with high quality for dysuria treatment which contains an active ingredient in constant content can be prepared at low price by using KMD-3213 of the crystal form α as an active ingredient.

As described above, hygroscopicities and stabilities of the crystal forms β and γ of KMD-3213 are the almost same as those of the crystal form α. The crystal form β, the crystal form γ or their mixture can be used together therewith for the oral solid medicament of the present invention as active ingredients, if its quality including the residual solvent is in an acceptable range. In such a case, it is not a problem that the oral solid medicament for dysuria treatment of the present invention contains the other crystal forms other than the crystal form α as active ingredients, and a mixture of the crystal form α and the other crystal forms can be used as an active ingredient of the oral solid medicament of the present invention.

The oral solid medicaments of the present invention can be prepared by the conventional pharmaceutical procedure. For example, capsules can be prepared by admixing the crystal form α of KMD-3213 or a mixture of the crystal form α and the other crystal forms, and excipients such as D-mannitol or lactose into water, kneading the mixture, sieving and drying to produce granules, and admixing the granules with lubricants such as magnesium stearate, and filling an appropriate capsule with the resulting mixture.

Tablets can be prepared by producing granules according to a similar manner to that of the above capsules, admixing lubricants such as magnesium stearate into the granules, punching the mixture into tablets by the conventional procedure and coating with an appropriate coating material.

Since KMD-3213 represented by the above formula (I) in the present invention is unstable for light relatively, the content of active ingredient decreases depending on preserved condition with the passage of time. Therefore, concerning capsules or tablets, capsules filled using a light shielding capsule or tablets coated by a coating material with a light shielding effect is preferable. As examples of the light shielding capsule or the coating material with a light shielding effect, a capsule containing titanium oxide or a coating material containing titanium oxide is the most preferable.

Up to the present, as for the indoline compound represented by the above formula (I) or its pharmaceutically acceptable salt, or pharmaceutically acceptable solvate thereof, and pharmaceutical compositions comprising as an active ingredient the same, these general dosage forms have been exemplified and these compositions are only described as producible according to general or known methods, for example, in the above Literature 1 or 2.

As described above, crystal polymorphism of KMD-3213 represented by the above formula (I), which is comprised as an active ingredient in the oral solid medicament of the present invention, has not investigated at all, and therefore, there are no description concerning what types of crystal forms exist, how to prepare them and what property they have. Furthermore, oral solid medicaments comprising as an active ingredient each crystal form of the indoline compound represented by the above formula (I) of the present invention are not reported nor suggested at all.

KMD-3213 represented by the above formula (I), which is comprised as an active ingredient in the oral solid medicament of the present invention, is a known compound and, for example, can be prepared by the procedure described in the above Literature 1.

KMD-3213 represented by the above formula (I) in the present invention exerts an α₁-AR blocking effect with less effect on blood pressure and is extremely useful as a therapeutic agent for dysuria caused by prostate hypertrophy etc. It is also expected that KMD-3213 represented by the above formula (I) in the present invention can be used as agents for dysuria associated with urethra organized obstruction except for prostate hypertrophy, such as urethra stricture, urethra calculus and prostate cancer, dysuria associated with disorder of urination control nerves and dysruria associated with urethra functional obstruction, which is not included in any dysuria as referred above, such as bladder cervix sclerosis, chronic prostatitis and unstable bladder etc.

Dysuria associated with disorder of urination control nerves means dysuria caused by disorder of control nerves in the urethra or the bladder, for example, encephalopathy such as disorder of cerebral blood vessel and brain tumor, disorder of spinal cord such as injury of spinal cord and disorder of peripheral nerves such as diabetes and lumbar region spine stenosis. These disorders may occur in both men and women and are generally called as neurogenic bladder.

Dysruria associated with urethra functional obstruction not accompanying urethra organized disorder and disorder of urination control nerves means dysuria caused by urination difficulty, bladder cervix blockage, urethra syndrome, detrusor muscle-sphincter muscle cooperation insufficiency, chronic cystitis, prostatodynia, Hinman syndrome, Fowler syndrome, psychogenic dysuria, drug-induced dysuria, aging and the like besides bladder cervix sclerosis, chronic prostatitis and unstable bladder. These disorders are generally called as lower urinary tract disorders.

Since the medicaments of the present invention have high precision of content of an active ingredient and good elution properties, they can exhibit the action of KMD-3213 represented by the above formula (I) in the present invention effectively. Accordingly, the medicaments of the present invention are extremely useful as agents for the treatment of dysuria associated with urethra organized obstruction such as prostate hypertrophy, urethra stricture, urethra calculus and prostate cancer; dysuria caused by disorder of urination control nerves, namely neurogenic bladder; and dysuria caused by urethra functional obstruction, namely lower urinary tract disorders.

In the case of using the above medicament of the present invention for a practical medical treatment, the dosage of the active ingredient is appropriately determined depending on the sex, age or body weight of the individual patient, the condition to be treated and the like, which is approximately within the range of from 1 to 50mg, preferably 4 to 20mg per day per adult human.

The medicaments of the present invention may comprise as a further active ingredient at least one member selected from a group of an α₁-AR blocker except for KMD-3213, an anticholinergic agent, an antiinflammatory agent and an antibacterial agent.

The medicaments of the present invention may be also used in combination with a medicament comprising as an active ingredient at least one member selected from a group of an α₁-AR blocking agent except for KMD-3213, an anticholinergic agent, a 5α-reductase inhibitor, a sex hormone agent, an antianxiety agent, a cholinergic agent, a cholinesterase inhibitor, an antiinflammatory agent and an antibacterial agent. In the present invention, a medicament for dysuria treatment which comprises combining a medicament comprising as an active ingredient a crystal for an oral solid medicament of KMD-3213 with a medicament comprising at least one member selected from a group of an α₁-AR blocking agent except for KMD-3213, an anticholinergic agent, a 5α-reductase inhibitor, a sex hormone agent, an antianxiety agent, a cholinergic agent, a cholinesterase inhibitor, an antiinflammatory agent and an antibacterial agent means a medicament comprising as an active ingredient KMD-3213 which is produced to be usable in combination with a medicament comprising at least one member selected from a group of an α₁-AR blocking agent except for KMD-3213, an anticholinergic agent, a 5α-reductase inhibitor, a sex hormone agent, an antianxiety agent, a cholinergic agent, a cholinesterase inhibitor, an antiinflammatory agent and an antibacterial agent, and a pharmaceutical kit composed of the combination.

In these situations, the contents of KMD-3213 represented by the above formula (I), and the contents of an α₁-AR blocking agent except for KMD-3213 represented by the above formula (I), an anticholinergic agent, a 5α-reductase inhibitor, a sex hormone agent, an antianxiety agent, a cholinergic agent, a cholinesterase inhibitor, an antiinflammatory agent and an antibacterial agent may be suitably reduced.

### EXAMPLE

The present invention is further illustrated in more detail by way of the following Reference Examples, Examples and Comparative Examples.

### Example 1

### Preparation of the crystal form α

To 1g of crude crystals of KMD-3213 was added 3mL of ethyl acetate, and the mixture was heated to dissolve. After insoluble materials were filtered off, the filtrate was allowed to stand at room temperature. After completion of precipitation of the resulting crystals, 10mL of ethyl acetate was added thereto. The resulting crystals were collected by filtration, and dried at 50°C for 16 hours in vacuo to give 930mg of the crystal form α.

### Example 2

### Preparation of the crystal form β

To 1g of crude crystals of KMD-3213 was added 0.4mL of methanol, and the mixture was heated to dissolve. After insoluble materials were filtered off, 20mL of petroleum ether was added thereto, and shook vigorously. The resulting crystals were collected by filtration, and dried at 50°C for 16 hours in vacuo to give 930mg of the crystal form β.

### Example 3

### Preparation of the crystal form γ

To 1g of crude crystals of KMD-3213 was added 4mL of toluene, and the mixture was heated to dissolve. After insoluble materials were filtered off, the filtrate was allowed to stand at room temperature. After completion of precipitation of the resulting crystals, 10mL of toluene was added thereto. The resulting crystals were collected by filtration, and dried at 50°C for 16 hours in vacuo to give 970mg of the crystal form γ.

### Test Example 1

### Stability test

Appearance and purity of each crystal obtained in Examples 1-3 and an amorphous obtained by freeze-drying a solution of KMD-3213 in dioxane were determined after allowing to stand under following conditions. As for appearance, it was determined by color and properties with the naked eye. Purity was determined by integral percentage method with liquid chromatography.

### The storage condition:

Condition 1: Allowing to stand at 40°C in the constant temperature device for 28 days
Condition 2: Allowing to stand at 60°C in the constant temperature device for 28 days
Condition 3: Allowing to stand at 80°C in the constant temperature device for 25 days
Condition 4: Allowing to stand at 40°C and relative humidity 75% in the constant temperature device for 28 days

### The liquid chromatography condition:

Detector: ultraviolet absorption spectrophotometer (wave length: 225 nm)
Column: Intertsil ODS-3 (GL Science, 5pm, 4.6 mm x 25 cm)
Column temperature: about 25°C
Mobile Phase: To 3.9g of sodium dihydrogen phosphate was added 2.5mL of diluted phosphoric acid solution (1 → 20) accurately, and water was added to make 1000mL of a solution accurately. A 5:2 mixed solution of the resulting solution and acetonitrile.
Flow rate: 1.0mL/min

As the result is shown in Table 1, the crystal form α was extremely stable in respect of purity and appearance.

**[Table 1]**

| Crystal | α | | β | | γ | | amorphous | |
|---|---|---|---|---|---|---|---|---|
| Item | Appearance | Purity (%) | Appearance | Purity (%) | Appearance | Purity (%) | Appearance | Purity (%) |
| Initial | White powder | 99.90 | Pale yellow powder | 99.83 | Pale yellow powder | 99.88 | White powder | 99.79 |
| Condition 1 | White powder | 99.84 | Pale yellow powder | 99.73 | Pale yellow powder | 99.85 | White powder | 99.66 |
| Condition 2 | White powder | 99.84 | Pale yello-wish brown powder | 99.57 | Pale yellow powder | 99.84 | Pale yellow powder | 99.56 |
| Condition 3 | Pale yellow powder | 99.58 | Pale brown powder | 98.42 | Pale yellow powder | 99.64 | Pale yello-wish brown powder | 99.53 |
| Condition 4 | White powder | 99.85 | Pale yellow powder | 99.69 | Pale yellow powder | 99.85 | White powder | 99.71 |

### Test Example 2

### Moisture absorption test

In the same manner as that of Test Example 1, the crystals obtained in Examples 1-3 and the amorphous were used for the test. Approximately 1 g of test substance was weighed accurately and put it into a sample vial. After the test substance was allowed to stand in an opening constant temperature and humidity device under following condition, their differences of weights were measured.

### The storage condition:

Condition 1: Allowing to stand at 25°C and 60% of relative humidity in the constant temperature and humidity device for 3days
Condition 2: Allowing to stand at 40°C and 75% of relative humidity in the constant temperature and humidity device for 3days

As the result is shown in Table 2, the crystal forms α, β and γ showed properties of absorbing moisture scarcely, and were stable compared with the amorphous.

**[Table 2]**

| crystal | Condition 1 | | Condition 2 | |
|---|---|---|---|---|
| | Initial weight (mg) | Change(%) | Initial weight (mg) | Change(%) |
| α | 947.78 | -0.01 | 1010.59 | -0.20 |
| β | 1000.51 | +0.07 | 1090.14 | -0.08 |
| γ | 992.79 | 0.00 | 993.04 | -0.19 |
| amorphous | 1001.09 | +1.61 | 1005.69 | +1.31 |

### Example 4

### Capsules 1

| | |
|---|---|
| Prescription: | |
| The crystal form α of KMD-3213 | 2.0 g |
| D-Mannitol | 134.4 g |
| Partially alpha starch (PCS) | 26.0 g |
| Partially alpha starch (Starch 1500) | 9.0 g |
| Magnesium stearate | 1.8 g |
| Sodium lauryl sulfate | 0.2 g |

According above prescription, 1000 capsules containing 2.0 mg of KMD-3213 in one capsule were formulated, by conventional method.

### Example 5

### Capsules 2

| | |
|---|---|
| Prescription: | |
| The crystal form α of KMD-3213 | 2.0 g |
| D-Mannitol | 134.4 g |
| Partially alpha starch (PCS) | 26.0 g |
| Partially alpha starch (Starch 1500) | 9.0 g |
| Magnesium stearate | 1.8 g |
| Sodium lauryl sulfate | 0.5 g |

According above prescription, 1000 capsules containing 2.0 mg of KMD-3213 in one capsule were formulated, by conventional method.

### Example 6

### Capsules 3

| | |
|---|---|
| Prescription: | |
| The crystal form α of KMD-3213 | 2.0 g |
| D-Mannitol | 134.4 g |
| Partially alpha starch (PCS) | 26.0 g |
| Partially alpha starch (Starch 1500) | 9.0 g |
| Magnesium stearate | 0.9 g |
| Sodium lauryl sulfate | 1.8 g |

According above prescription, 1000 capsules containing 2.0 mg of KMD-3213 in one capsule were formulated, by conventional method.

### Example 7

### Capsules 4

| | |
|---|---|
| Prescription: | |
| The crystal form α of KMD-3213 | 2.0 g |
| D-Mannitol | 134.4 g |
| Partially alpha starch (PCS) | 26.0 g |
| Partially alpha starch (Starch 1500) | 9.0 g |
| Magnesium stearate | 1.8 g |
| Sodium lauryl sulfate | 1.8 g |

According above prescription, 1000 capsules containing 2.0 mg of KMD-3213 in one capsule were formulated, by conventional method.

### Example 8

### Capsules 5

| | |
|---|---|
| Prescription: | |
| The crystal form α of KMD-3213 | 4.0 g |
| D-Mannitol | 132.4 g |
| Partially alpha starch (PCS) | 26.0 g |
| Partially alpha starch (Starch 1500) | 9.0 g |
| Magnesium stearate | 1.8 g |
| Sodium lauryl sulfate | 1.8 g |

According above prescription, 1000 capsules containing 4.0 mg of KMD-3213 in one capsule were formulated, by conventional method.

### Example 9

### Tablets

| | |
|---|---|
| Prescription: | |
| The crystal form α of KMD-3213 | 4.0 g |
| D-Mannitol | 117.0 g |
| Corn starch | 7.0 g |
| L-HPC | 7.0 g |
| HPC-SL | 4.0 g |
| Magnesium stearate | 1.0 g |

According above prescription, 1000 tablets containing 4.0 mg of KMD-3213 in one tablet were formulated, by conventional method.

### INDUSTRIAL APPLICABILITY

In the present invention the crystal form α, β and γ of KMD-3213 represented by the above formula (I) show properties of absorbing moisture scarcely, and stability. Since the crystal form α is very stable and has no problem in respect of industrial preparation and can be prepared regularly, easily and in a large scale, the crystal form α is the most preferable crystal for the oral solid medicament. Furthermore, the crystal form α, β and γ show almost same even hygroscopocity and stability of lowering of purity other than stability of appearance, so the mixture of the crystal form α and other crystal forms can be used for preparation of the oral solid medicament of the present invention as active ingredients.

Accordingly, the oral solid medicament for dysuria treatment can be prepared by containing the crystal form α or the mixture of crystal form α and other crystal forms as active ingredients.

Especially, the oral solid medicament of the present invention containing the crystal form α as an active ingredient shows the content of the active ingredient regularly, good stability and small lowering of the content in preservation, and it is extremely a superior oral solid medicament for dysuria treatment.

## Claims

1. A crystal for an oral solid medicament of an indoline compound represented by the formula: 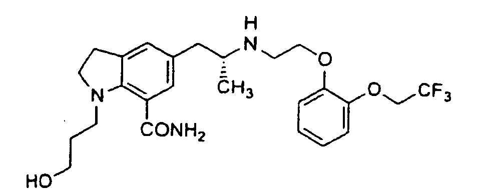 which shows an X-ray powder diffraction pattern **characterized by** main peaks of 5.5° ± 0.2°, 6.1° ± 0.2°, 9.8° ± 0.2°, 11.1° ± 0.2°, 12.2° ± 0.2°, 16.4° ± 0.2°, 19.7° ± 0.2° and 20.0° ± 0.2° as 2θ.

2. An oral solid medicament for dysuria treatment comprising as an active ingredient the crystal as claimed in claim 1.

3. A medicament as claimed in claim 2, which comprises as a further active ingredient at least one member selected from a group of an α₁-adrenoceptor blocking agent except for the indoline compound represented by the formula: 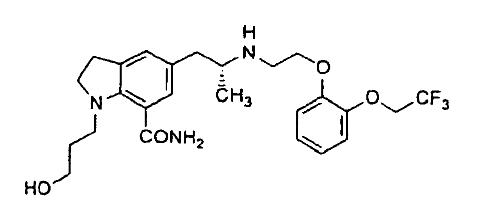 an anticholinergic agent, a 5α-reductase inhibitor, a sex hormone agent, an antianxiety agent, a cholinergic agent, a cholinesterase inhibitor, an antiinflammatory agent and an antibacterial agent.

4. A medicament as claimed in claim 2 or 3, wherein the dosage form is capsules or tablets.

5. A medicament as claimed in claim 4, wherein the capsules is filled in a light shielding capsule or the tablets is coated by a coating material with a light shielding effect.

6. A medicament as claimed in claim 5, wherein the light shielding capsules is a capsule containing titanium oxide.

7. A medicament as claimed in claim 5, wherein the coating material with a light shielding effect is a coating material containing titanium oxide.

8. A medicament for dysuria treatment which comprises combining a medicament as claimed in claim 2 with a medicament comprising as an active ingredient at least one member selected from a group of an α₁-adrenoceptor blocking agent except for the indoline compound represented by the formula: 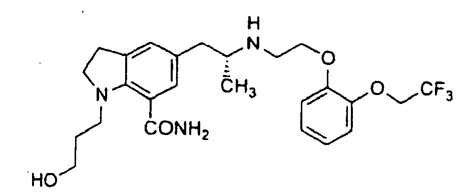 an anticholinergic agent, a 5α-reductase inhibitor, a sex hormone agent, an antianxiety agent, a cholinergic agent, a cholinesterase inhibitor, an antiinflammatory agent and an antibacterial agent.

9. A medicament as claimed in any one of claims 2 to 8, wherein the dysuria is caused by dysuria associated with urethra organized obstruction, dysuria associated with a disorder of urination control nerves or dysuria associated with urethra functional obstruction.

10. A medicament as claimed in any one of claims 2 to 8, wherein the dysuria is caused by prostate hypertrophy, urethra stricture, urethra calculus, prostate cancer, neurogenic bladder or lower urinary tract disorders.

## Patentansprüche

1. Kristall für ein orales festes Medikament aus einer Indolin-Verbindung, wiedergegeben durch die Formel: 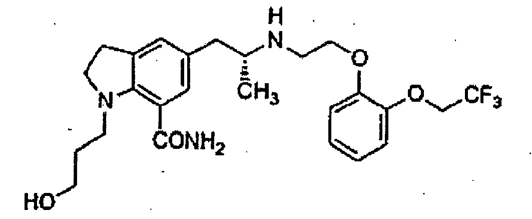 welcher ein Röntgenpulverdiffraktometrie-Muster zeigt, das durch Hauptpeaks bei 5,5° ± 0,2°, 6,1° ± 0,2°, 9,8°± 0,2°, 11,1°± 0,2°, 12,2°± 0,2°, 16,4°± 0,2°, 19,7°± 0,2° und 20,0°± 0,2° als 2 θ gekennzeichnet ist.

2. Orales festes Medikament zur Dysurie-Behandlung, welches als einen wirksamen Bestandteil den Kristall nach Anspruch 1 umfasst.

3. Medikament nach Anspruch 2, welches als einen weiteren wirksamen Bestandteil wenigstens ein Mitglied umfasst, das ausgewählt ist aus einer Gruppe eines α1-Adrenozeptor-blockierenden Mittels, mit Ausnahme der durch die Formel 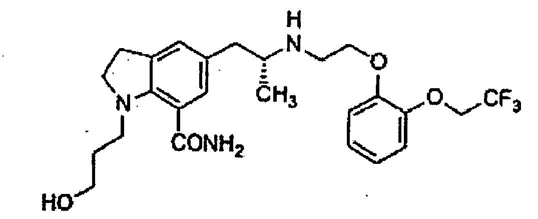 wiedergegebenen Indol-Verbindung, eines anticholinergen Mittels, eines 5α-Reduktase-Inhibitors, eines Sexualhormon-Mittels, eines Antiangstmittels, eines cholinergen Mittels, eines Cholinesterase-Inhibitors, eines antiinflammatorischen Mittels und eines antibakteriellen Mittels.

4. Medikament nach Anspruch 2 oder 3, worin die Dosierungsform Kapseln oder Tabletten sind.

5. Medikament nach Anspruch 4, worin die Kapsel in eine Licht-abschirmende Kapsel abgefüllt ist, oder die Tablette mit einem Beschichtungsmaterial mit Licht-abschirmender Wirkung beschichtet ist.

6. Medikament nach Anspruch 5, worin die Licht-abschirmende Kapsel eine Kapsel ist, die Titanoxid enthält.

7. Medikament nach Anspruch 5, worin das Beschichtungsmaterial mit Licht-abschirmender Wirkung ein Beschichtungsmaterial ist, das Titanoxid enthält.

8. Medikament zur Dysurie-Behandlung, welches eine Kombination eines Medikaments nach Anspruch 2 mit einem Medikament umfasst, das als einen wirksamen Bestandteil wenigstens ein Mitglied umfasst, das ausgewählt ist aus einer Gruppe eines α1-Adrenozeptor-blockierenden Mittels, mit Ausnahme der durch die Formel 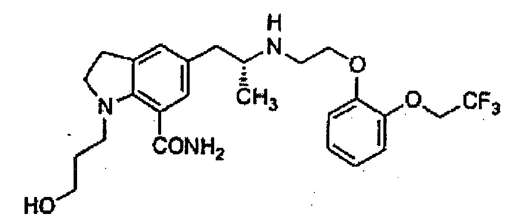 wiedergegebenen Indol-Verbindung, eines anticholinergen Mittels, eines 5α-Reduktase-Inhibitors, eines Sexualhormon-Mittels, eines Antiangstmittels, eines cholinergen Mittels, eines Cholinesterase-Inhibitors, eines antiinflammatorischen Mittels und eines antibakteriellen Mittels.

9. Medikament nach einem der Ansprüche 2 bis 8, worin die Dysurie durch Dysurie im Zusammenhang mit organisiertem Harnröhrenverschluss, Dysurie im Zusammenhang mit einer Störung der Harnentleerungskontrollnerven oder Dysurie im Zusammenhang mit funktionellem Harnröhrenverschluss hervorgerufen ist.

10. Medikament nach einem der Ansprüche 2 bis 8, worin die Dysurie durch Prostatahypertrophie, Harnröhrenstriktur, Harnröhrenstein, Prostatakrebs, neurogene Blase oder Störungen der unteren Harnwege hervorgerufen ist.

## Revendications

1. Cristal pour un médicament solide oral constitué d'un dérivé d'indoline représenté par la formule : 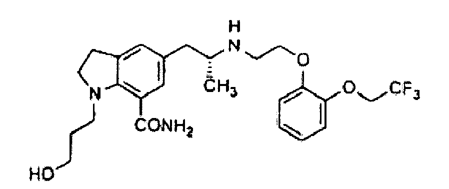 qui présente un diagramme de diffraction des rayons X sur poudre **caractérisé par** des pics principaux à 5,5 ° ± 0,2 °, 6,1 ° ± 0,2 °, 9,8 ° ± 0,2 °, 11,1 ° ± 0,2 °, 12,2 ° ± 0,2 °, 16,4 ° ± 0,2 °, 19,7 ° ± 0,2 ° et 20,0 ° ± 0,2 ° sous forme de 2θ.

2. Médicament solide oral pour le traitement de la dysurie, comprenant comme ingrédient actif le cristal suivant la revendication 1.

3. Médicament suivant la revendication 2, qui comprend comme ingrédient actif supplémentaire au moins un membre choisi dans le groupe comprenant un agent de blocage des adrénorécepteurs α₁, à l'exception du dérivé d'indoline représenté par la formule : 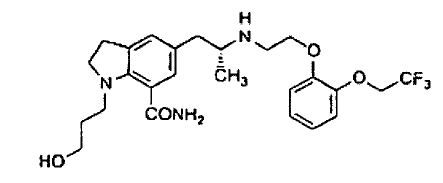 un agent anticholinergique, un inhibiteur de 5α-réductase, un agent du type hormone sexuelle, un anxiolytique, un agent cholinergique, un inhibiteur de cholinestérase, un agent anti-inflammatoire et un agent antibactérien.

4. Médicament suivant la revendication 2 ou 3, dans lequel la forme posologique consiste en capsules ou comprimés.

5. Médicament suivant la revendication 4, dans lequel la capsule est remplie en utilisant une capsule faisant écran à la lumière ou bien le comprimé est enrobé avec une matière d'enrobage faisant écran à la lumière.

6. Médicament suivant la revendication 5, dans lequel la capsule faisant écran à la lumière est une capsule contenant de l'oxyde de titane.

7. Médicament suivant la revendication 5, dans lequel la matière d'enrobage faisant écran à la lumière est une matière d'enrobage contenant de l'oxyde de titane.

8. Médicament pour le traitement de la dysurie, qui comprend l'association d'un médicament suivant la revendication 2 avec un médicament comprenant comme ingrédient actif au moins un élément choisi dans le groupe comprenant un agent de blocage des adrénorécepteurs α₁, à l'exception du dérivé d'indoline, représenté par la formule : 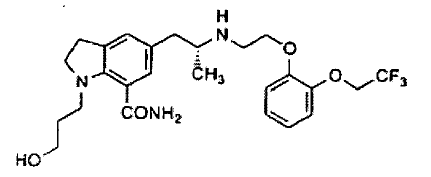 un agent anticholinergique, un inhibiteur de 5α-réductase, un agent du type hormone sexuelle, un anxiolytique, un agent cholinergique, un inhibiteur de cholinestérase, un agent anti-inflammatoire et un agent antibactérien.

9. Médicament suivant l'une quelconque des revendications 2 à 8, dans lequel la dysurie est provoquée par la dysurie associée à une obstruction organisée de l'urètre, la dysurie associée à un trouble des nerfs commandant la miction ou la dysurie associée à une obstruction fonctionnelle de l'urètre.

10. Médicament suivant l'une quelconque des revendications 2 à 8, dans lequel la dysurie est provoquée par une hypertrophie de la prostate, une striction de l'urètre, un calcul urétral, le cancer de la prostate, une vessie neurogène ou des troubles dans la partie inférieure du tractus urinaire.
